Europäisches Patentamt
European Patent Office
Office européen des brevets

(19)

(11) Publication number: **0 596 112 A1**

(12) **EUROPEAN PATENT APPLICATION published in accordance with Art. 158(3) EPC**


(21) Application number: **91920688.8**

(22) Date of filing: **27.11.91**

(86) International application number: **PCT/JP91/01629**

(87) International publication number: **WO 92/12988 (06.08.92 92/21)**

(51) Int. Cl.5: **C07H 17/08**, C12N 1/20, C12P 19/62, //A61K31/71, (C12P19/62,C12R1:01)

(30) Priority: **18.01.91 JP 78144/91**

(43) Date of publication of application: **11.05.94 Bulletin 94/19**

(84) Designated Contracting States: **AT BE CH DE DK ES FR GB GR IT LI LU NL SE**



(71) Applicant: **YAMANOUCHI PHARMACEUTICAL CO. LTD.**
**No. 3-11 Nihonbashi-Honcho, 2-chome**
**Chuo-ku**
**Tokyo 103(JP)**
Applicant: **SHANGHAI INSTITUTE OF MATERIA MEDICA ACADEMIA SHINICA**
**319 Yue-Yang Road**
**Shanghai(CN)**

(72) Inventor: **MORIOKA, Motoo**
**12-1, Atago 3-chome**
**Niiza-shi, Saitama 352(JP)**
Inventor: **TAKAHASHI, Isao**
**500-5, Ooaza Minamitajima**
**Kawagoe-shi, Saitama 356(JP)**
Inventor: **SUZUKI, Kenichi**
**1-67, Futatsuya**
**Kitamoto-shi, Saitama 236(JP)**
Inventor: **NAGAI, Koji**
**16-1, Hasune 3-chome,**
**Itabashi-ku**
**Tokyo 174(JP)**
Inventor: **YAMAGUCHI, Hiroshi**
**136-51-10-205, Nara-cho**
**Omiya-shi, Saitama 331(JP)**
Inventor: **SASAKI, Toshio**
**22-12-504, Buzo 5-chome**
**Urawa-shi, Saitama 336(JP)**
Inventor: **SHIBAZAKI, Mitsuyoshi**
**16-1, Hasune 3-chome,**
**Itabashi-ku**
**Tokyo 174(JP)**
Inventor: **HIRAMOTO, Masashi**
**134, Gumyoji-cho,**
**Minami-ku**
**Yokohama-shi, Kanagawa 232(JP)**
Inventor: **TAKEBAYASHI, Yukihiro**
**3-2, Wakamiya 3-chome**
**Ichikawa-shi, Chiba 272(JP)**
Inventor: **SUSAKI, Kiyoshi**
**11-30, Fujimi 1-chome**
**Ageo-shi, Saitama 362(JP)**
Inventor: **LIANG, Shu Fang, Shanghai Institute of Materia**
**Medica Academia Shinica,**
**319, Yue-Yang Road**
**Shanghai 200031(CN)**
Inventor: **WANG, Fu Jin, Shanghai Institute of Materia**
**Medica Academia Shinica,**
**319, Yue-Yang Road**
**Shanghai 200031(CN)**
Inventor: **ZANG, Wei Wen, Shanghai Institute of Materia**
**Medica Academia Shinica,**
**319, Yue-Yang Road**
**Shanghai 200031(CN)**
Inventor: **LIU, Jia Wei, Shanghai Institute of Materia**
**Medica Academia Shinica,**
**319, Yue-Yang Road**
**Shanghai 200031(CN)**

(74) Representative: **Geering, Keith Edwin**
**REDDIE & GROSE**
**16 Theobalds Road**





Rank Xerox (UK) Business Services
(3.10/3.09/3.3.4)

London WC1X 8PL (GB)

(54) **NOVEL MACROLIDE COMPOUND AND PRODUCTION THEREOF.**

(57) A novel macrolide compound represented by general formula (I), a process for the production thereof and a microorganism belonging to the genus *Kibdelosporangium* which produces said macrolide compound. In formula (I), $R^1$ represents isovaleryloxy or hydroxy; $R^2$ represents methyl or hydroxymethyl; and $R^3$ represents methyl or hydrogen. This compound is obtained from the medium for culturing the microorganism, and is useful as medicine, especially an antibacterial agent.

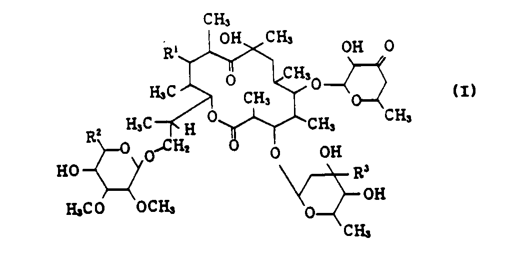

(I)

## Technical Field

The present invention relates to a macrolide compound useful as a medicament, particularly as an antibacterial agent, a fermentative process for producing the compound and a novel microorganism to be used in the process.

In the course of their exploratory research into the metabolites of naturally-occurring microorganisms, the inventors of the present invention discovered a novel macrolide antibiotic substance having a strong antimicrobial activity in the culture of a macrolide antibiotic-producing strain of microorganism of the genus Kibdelosporangium and isolated the substance, whereby the present invention was brought into being.

## Background Art

The macrolide compounds produced by microorganisms are generally known to belong to either a class of basic macrolides or a class of neutral macrolides. As compounds belonging to the class of basic macrolides, there are known 12-membered ring macrolides such as methymycin, neomethymycin, etc., 14-membered ring macrolides such as erythromycin, oleandmycin, pikromycin, megalomicin, etc. and 16-membered ring macrolides such as josamycin, leucomycin, spiramycin, carbomycin, tylosin, angolamycin, mycinamicin, rosamicin, juvenimicin and so on. As neutral macrolides, there are known 14-membered ring macrolides such as lankamycin, kujimycin, etc. and 16-membered ring macrolides such as chalkomycin, aldgamycin and so on. As a compound belonging to the class of 14-membered ring macrolides, J. Am. Chem. Sol. 102 (10), 3605-3608 (1980) describes 23672RP, a metabolic product of Streptomyces chryseus DS12370 (NRRL3892). While some of these macrolides have been utilized clinically as remarkably useful antibacterials, the macrolide compound of the present invention differentiates itself clearly from any of these known antibacterials structurally, for example in the sugar moieties and in the radicals substituting the 14-membered lactone structure. Furthermore, it is a novel 14-membered ring macrolide compound having high antibacterial activity against gram-positive bacteria. Therefore, the present invention provides a novel macrolide compound having very satisfactory antibacterial activity. The present invention further provides a novel method for producing the novel macrolide compound. Furthermore, the invention provides a new species of microorganism capable of producing the macrolide compound.

## Disclosure of Invention

Thus, the present invention is directed to a novel macrolide compound of the following chemical formula (I).

(I)

(wherein $R^1$ represents isovaleryloxy

$$\left( \begin{matrix} CH_3 \\ CH_3 \end{matrix} > CHCH_2\overset{\overset{O}{\|}}{C}O- \right)$$

or hydroxy; $R^2$ represents methyl or hydroxymethyl; $R^3$ represents methyl or hydrogen; the same definitions apply hereinafter).

The present invention is further directed to a novel method for producing the above novel macrolide compound which comprises culturing a strain of microorganism capable of producing the macrolide compound (I) in a culture medium to let the strain produce said compound in the resulting culture and harvesting the compound therefrom.

The present invention still further provides a novel species of microorganism which has an ability to produce the macrolide compound (I).

## Brief Description of Drawings

Fig. 1 shows an ultraviolet absorption spectrum of YL-02107Q substance in methanol.
Fig. 2 shows an infrared absorption spectrum of YL-02107Q substance.
Fig. 3 shows an $^1$H-NMR spectrum of YL-02107Q substance.
Fig. 4 shows a $^{13}$C-NMR spectrum of YL-02107Q substance.
Fig. 5 shows an ultraviolet absorption spectrum of YL-02107Q-B through D substance in methanol.
Fig. 6 shows an infrared absorption spectrum of YL-02107Q-B substance.
Fig. 7 shows an infrared absorption spectrum of YL-02107Q-C substance.
Fig. 8 shows an infrared absorption spectrum of YL-02107Q-D substance.
Fig. 9 shows an $^1$H-NMR spectrum of YL-02107Q-B substance.
Fig. 10 shows an $^1$H-NMR spectrum of YL-02107Q-C substance.
Fig. 11 shows an $^1$H-NMR spectrum of YL-02107Q-D substance.
Fig. 12 shows a $^{13}$C-NMR spectrum of YL-02107Q-B substance.
Fig. 13 shows a $^{13}$C-NMR spectrum of YL-02107Q-C substance.
Fig. 14 shows a $^{13}$C-NMR spectrum of YL-02107Q-D substance.

## Best Mode for Practicing the Invention

The strain of microorganism which is able to produce the compound of this invention is a microorganism isolated from a soil sample obtained in Chichihaerh of the People's Republic of China and having the following microbiological characters.

### (1) Morphology

Producing well-developed, branched substrate mycelia on various organic and inorganic media, with the hyphae measuring about 0.4 µm in width and depending on media, being slightly fragmented. The aerial mycelium appears gray-white and is elongated and linear or gently curved, branching out irregularly. Not less than 50 spores are observed in a chain, with spores being cylindrical, measuring 0.4 - 0.6 µm x 1.5 - 2.5 µm, and each having a smooth surface. On certain media such as oatmeal agar, many sporangium-like structures are observed in the aerial mycelium. These structures are subspherical in shape and have creased surfaces, measuring 5 - 15 µm in size and without evidence of spores. Such organs as sclerotium, motile element, whorl, etc. are not observed.

### (2) Cultural characteristics on agar media

The characteristics of the strain on various agar media are as follows. Unless otherwise indicated, the following characterizations were made in the conventional manner after 21 days of culture at 27°C. The color indications are according to the color standard (Color Institute of Japan).

Table 1: Cultural characteristics of YL-02107Q strain on agar media

| Medium | | |
|---|---|---|
| Sucrose nitrate agar | G | Moderate |
| | A | Moderate, gray-white - tan-white |
| | R | Light yellow-tan |
| | S | None |
| Glucose asparagine agar | G | Moderate |
| | A | Moderate, gray-white |
| | R | Light yellow-tan |
| | S | None |
| Glycerin asparagine agar (ISP-5) | G | Rather poor |
| | A | Rather sparse, bright gray |
| | R | Light yellow-tan |
| | S | None |
| Starch inorganic salt agar (ISP-4) | G | Good |
| | A | Abundant, gray-white |
| | R | Light yellow-tan - yellow-tan |
| | S | None |
| Tyrosine agar (ISP-7) | G | Moderate |
| | A | Moderate, gray-white |
| | R | Light yellow-tan |
| | S | None |
| Nutrient agar | G | Moderate |
| | A | Moderate, gray-white |
| | R | Light yellow-tan - yellow tan |
| | S | None |
| Yeast malt agar (ISP-2) | G | Good |
| | A | Abundant, gray-white |
| | R | Light yellow-tan - yellow tan |
| | S | None |

| | | |
|---|---|---|
| Oatmeal agar (ISP-3) | G | Good |
| | A | Abundant, gray-white |
| | R | Light yellow-tan - Yellow tan |
| | S | None |
| Benett agar | G | Moderate |
| | A | Moderate, gray-white |
| | R | Light yellow-tan - yellow-tan |
| | S | None |
| Peptone yeast iron agar (ISP-6) | G | Moderate |
| | A | Abundant, bright gray |
| | R | Dark yellow-tan |
| | S | None |

(Note) G: growth

A: aerial mycelium and its color

R: Reverse color

S: Soluble pigments

(3) Physiological characteristics

Table 2

| Physiological characteristics of YL-02107Q strain | | |
|---|---|---|
| 1) | Temperature range for growth<br>Optimum temperature for growth | 15-45 °C<br>27-33 °C |
| 2) | Liquefication of gelatin<br>Simple gelatin (20 °C)<br>Glucose peptone gelatin (28 °C) | <br>Negative<br>Positive |
| 3) | Coagulation of skim milk<br>Peptonization of skim milk | Negative<br>Positive |
| 4)<br>5) | Reduction of nitrate<br>Hydrolysis of starch | Positive<br>Positive |
| 6) | Production of melanoid pigment<br>Tryptone yeast extract broth<br>Tyrosine agar<br>Peptone yeast iron agar | <br>Negative<br>Negative<br>Negative |
| 7) | Resistance to NaCl | ≦ 6% |
| (Note) The temperature range for growth was determined by culturing the strain at various temperatures (5, 10, 15, 20, 24, 27, 30, 33, 37, 40, 45, 50 °C) for 7 - 21 days and the action on milk was investigated by 3-21 days of observation at 37 °C. Unless otherwise indicated, the other results were findings after 2 weeks of incubation at 27 °C. | | |

(4) Utilization of carbon sources (Pridham-Gottlieb agar, 27 ° C)

Table 3

| Carbohydrate utilization spectrum of YL-02107Q strain | | | |
|---|---|---|---|
| L-Arabinose -<br>D-Mannitol +<br>Trehalose +<br>Sucrose +<br>Lactose - | Raffinose -<br>Salicin -<br>D-Fructose +<br>Melibiose -<br>Xanthine - | Maltose +<br>D-Glucose +<br>Mannose +<br>Dextrin +<br>Rhamnose + | D-Xylose +<br>Starch +<br>Glycerin +<br>Inositol +<br>D-Galactose + |
| (Note) + : growth -: no growth | | | |

5. Chemical analysis of cellular components

The acid hydrolyzate and cell wall of this strain were analyzed by the method of Lechvalier et al. (Lechvalier, M.P. et al; pp. 277-238 in Dietz, A. et al. ed., Actinomycete Taxonomy, SIM Special Publication No. 6, 1980). As shown in Table 4, the cell wall type was IV-A.

Table 4

| Chemical analysis of the YL-02107Q strain cell | |
|---|---|
| Cell wall constituent amino acids | Meso-diaminopimellic acid, alanine, glutamic acid |
| Total cell reducing sugar | Arabinose, galactose |
| Main menaquinone | MK-9 ($H_4$) |

The above characteristics may be summarized as follows. This YL-02107Q strain produces abundant aerial mycelium from substrate mycelium which extends into agar medium and forms spore chains in some of the aerial hyphae. The substrate mycelium may show slight fragmentation depending on media. The spore is rod-shaped, with a smooth surface ornamentation, and no motility is observed. A large number of sporangium-like structures are formed in the aerial mycelium but no spore is observed. Chemical analysis of the cell constituents revealed that the cell wall type was IV and the dominant menaquinone was MK-9 ($H_4$).

Literature searches for bacteria having similar characteristics revealed that in morphology and chemical analysis this strain was substantially identical to species of the genus Kibdelosporangium proposed by Shearer et al. in 1986. Therefore, the strain was regarded as an organism belonging to this genus. Accordingly, the various characteristics of this strain were compared with the reported characteristics of Kibdelosporangium aridum, which is the only species so far reported in this genus. As a result, it was found that K. aridum is different from the present strain in that it produces melanoid pigments, is negative in the reduction of nitrates and hydrolysis of starch, and produces a glycopeptide antibiotic. Therefore, the strain was identified to be a new species of the genus Kibdelosporangium and named Kibdelosporangium sp. YL-02107Q. This strain has been deposited with the following type culture collection.

(A) Name and address of the culture collection

Name: Fermentation Research Institute, Agency of Industrial Science and Technology, Ministry of International Trade and Industry

Address: 1-3, Higashi 1-chome, Tsukuba-shi, Ibaraki Prefecture, Japan (Postal Code: 305)

(B) Date of deposit (original deposit): May 19, 1990

(C) Accession number: FERM BP-3606

While this microorganism is ready to undergo mutation, whether artificial or spontaneous, the term "Kibdelosporangium sp. YL-02107Q" is used herein to mean not only the actinomycete isolated from the natural kingdom but also those strains which are derived from the actinomycete by a mutagenic treatment with ultraviolet light, X-rays or a chemical mutagen as well as spontaneous mutants and derivatives.

The method for producing the novel macrolide antibiotic compound in accordance with the present invention can be carried into practice as follows. Thus, Kibdelosporangium sp. YL-02107Q, which is the

producer of the compound, is inoculated into a culture medium containing nutrients and allowed to grow under aerobic conditions to obtain a culture containing the novel macrolide compound of the invention. The nutrients may be those known and used as nutrients for actinomycetes. For example, such inorganic or organic nitrogen sources as peptone, meat extract, corn steep liquor, cottonseed meal, peanut flour, soybean meal, yeast extract, NZ-amine, casein hydrolysate, fish meal, sodium nitrate, ammonium nitrate, etc., and such carbon sources as various carbohydrates, e.g. commercial molasses, starch, dextrin, sucrose, glucose, maltose, fructose, xylose, rhamnose, mannitol, glycerol, etc., and various fats can be employed. Where necessary, metal salts such as the sulfates, hydrochlorides, nitrates, phosphates, carbonates, etc. of Na, K, Mg, Ca, Zn, Fe, etc. can be added. In addition, antibiotic production promoters and antifoams such as valine, leucine, isoleucine, threonine, phenylalanine, tryptophan, methionine, lysine, arginine, and other amino acids which are well known, methyl oleate, lard oil, silicone oil, surfactants, etc. can also be added where necessary. Aside from them, any substance that this producer strain can utilize and is contributory to the production of the novel macrolide compound of the invention can be employed. The cultural method may be similar to that used generally for the production of antibiotics, and whichever of solid culture and liquid culture can be utilized. In the case of liquid culture, any of stationary culture, spinner culture, shake culture, etc. can be utilized, although aerobic spinner culture is particularly advantageous. The incubation temperature can be varied as desired within the range which permits growth of the producer strain and its production of the compound of the invention, namely the range of 15°C to 40°C, but the range of about 25°C to 32°C is preferred. The pH of the medium can be freely chosen within the range of 4 to 9 but is preferably pH 6 to 8. The incubation time varies with various conditions and may range from 10 to 168 hours. However, it is usual that the highest titer of the compound in the culture is attained in about 24 to 120 hours. The object compound can be harvested from the culture by the extraction, isolation and purification procedures applied commonly to microbial metabolites. Thus, the culture as such or its filtrate separated by centrifugation or filtration using a filter aid is treated with a water-immiscible organic solvent such as ethyl acetate, chloroform, benzene or toluene to extract the object compound. Alternatively, the culture filtrate is contacted with a suitable carrier to adsorb the object compound from the culture filtrate and, then, the compound is eluted with a suitable solvent to extract the object compound. To be specific, the object compound is adsorbed by contacting the culture filtrate with a porous adsorbent resin such as Diaion HP-20, Amberlite XAD-2, Diaion SP-900 or Diaion CHP-20P. Then, the object compound is eluted with a mixture of an organic solvent, e.g. methanol, ethanol, acetone or acetonitrile, and water. The ratio of the solvent should of course be such that the object compound can be eluted with the highest efficiency. Thus, by increasing the ratio of the solvent from a low concentration either stepwise or continuously to a high concentration, an fraction richer in the object compound can be obtained. Where the extraction with an organic solvent such as ethyl acetate or chloroform is performed, the extract fraction containing the object compound can be obtained by adding such a solvent to the filtrate of the culture, shaking the mixture to extract the object compound into the solvent and washing the extract serially with an acidic aqueous solution, e.g. diluted hydrochloric acid, or an alkaline aqueous solution, e.g. a dilute aqueous solution of sodium hydrogen carbonate.

The fraction containing the object compound as obtained by the above procedure can be further purified by the conventional adsorptive procedure, for example by column chromatography using activated carbon, alumina, silica gel, cellulose or the like as a carrier or by high performance liquid chromatography using a silica gel ODS reversed phase column or the like.

Example 1

A medium (pH 7.0) containing 2.0% of glucose, 2.0% of potato starch, 0.5% of yeast extract, 0.5% of polypeptone and 0.4% of calcium carbonate was prepared. This medium was distributed, in 60 ml portions, into 500 ml conical flasks and sterilized at 120°C for 20 minutes to provide seed media. These seed media were inoculated with the hyphae picked from a good growth of Kibdelosporangium sp. YL-02107Q on Benett agar and shake culture was carried out at 28°C for 48 hours to provide a seed culture. Then, for use as production media, the same medium as above was distributed and sterilized in exactly the same manner as above. These media were inoculated with the above seed culture at the level of 3% and incubated at 28°C for 72 hours. To 5 ℓ of the resulting culture was added Radiolite #600 (Showa Chemical Industries, Ltd.) and the mixture was stirred and filtered to provide 3.8 ℓ of culture filtrate. To this filtrate (pH 8.07) was added 2 ℓ of ethyl acetate and the resulting mixture was shaken well to extract the object compound YL-02107Q substance. This extract gave an inhibition zone with a diameter of 16.1 mm in the agar plate assay using a paper disk 8 mm in diameter (Toyo Seisakusho, Ltd.) and, as the test organism, Bacillus subtilis ATCC 6633. This extract was then dried over mirabilite, concentrated to dryness under reduced pressure

and dried in a vacuum desiccator overnight to provide 0.81 g of the object compound YL-02107Q substance as a crude solid. This crude solid was dissolved in a small amount of chloroform and applied to a 2.7 cm x 60 cm column of silica gel (Wakogel C-300, Wako Pure Chemical Industries, Ltd.) and elution was carried out with chloroform-methanol (100:1). The eluate was collected in 17 g fractions using a fraction collector and each fraction was tested for activity by the agar disk assay using Bacillus subtilis ATCC 6633. For the confirmation of the object compound YL-02107Q substance, thin-layer chromatography was performed using a silica gel thin-layer plate (Kieselgel 60 $F_{254}$, 20 cm x 20 cm, Merck Co.) and, as the developer, chloroform-methanol (90:10) and the object compound YL-02107Q substance (Rf = 0.43) was detected by sulfuric acid color reaction (sprayed with 10% sulfuric acid and heated at 110°C for 20 minutes) and bioautography using an agar plate of Bacillus subtilis ATCC 6633. It was found that the object compound had been eluted in fraction No. 131 through fraction No. 195. These fractions were pooled, concentrated to dryness under reduced pressure and dried in a vacuum desiccator overnight to provide 45 mg of crude YL-02107Q substance. For further purification, a solution of the crude YL-02107Q substance in a small amount of ethyl acetate was applied linearly to a silica gel thin-layer plate (kieselgel 60 $F_{254}$, 20 cm x 20 cm, Merck Co.) at a level 2 cm from the bottom edge and developed with chloroform-methanol (90:10). The thin-layer plate was then dried in the air and sprayed with distilled water. The spot of YL-02107Q substance (Rf = 0.43), which appeared as a white band, was marked off and after air-drying, the silica gel was scraped off from the spot and eluted with the same developer as above. YL-02107Q substance in this eluate was found to give a substantially single spot by the above thin-layer chromatography. Therefore, this eluate was concentrated to dryness at 40°C under reduced pressure and dried overnight in a vacuum desiccator to provide 22 mg of a white powder of YL-02107Q substance with a purity of 90%.

Example 2

The preparation of the seed culture medium and the cultivation of the seed culture were carried out in exactly the same manner as in Example 1. The two-stage cultural method was employed. The first and second stages were performed for 2 days each to finally provide 400 ml of a seed culture. For production culture, a 30 ℓ tank fermenter was charged with 18 ℓ of a medium (pH 7.0) containing 1.0% of glucose, 2.0% of potato starch, 0.5% of soybean meal, 0.5% of feather meal and 0.4% of calcium carbonate, which was then sterilized at 120°C for 30 minutes. This fermenter was inoculated with the total amount, 400 ml, of the seed culture and incubated at 200 rpm for 3 days with an aeration rate of 30 ℓ per minute. Thereupon, the antibacterial activity of the object compound YL-02107Q substance against Bacillus subtilis ATCC 6633 became maximal. To this culture was added Radiolite #600 (Showa Chemical Industries, Ltd.) and the mixture was stirred and filtered. The resulting filtrate (pH 8.2) was adjusted to pH 7.0 with hydrochloric acid and applied to a column prepared by packing a glass tube having an outer diameter of 8 cm and a height of 30 cm with 1 ℓ of Diaion HP-20 to adsorb the object compound. The column was then rinsed well with 10 ℓ of distilled water and further washed with 15 ℓ of 25% acetone-water and finally elution was carried out with 15 ℓ of 70% acetone-water. This eluate was concentrated to 200 ml under reduced pressure to provide an extract containing the object compound YL-02107Q substance. This extract was adjusted to pH 7.0 and shaken well with 500 ml of ethyl acetate. After phase separation, the water layer was discarded. The extract thus obtained was thoroughly dehydrated by addition of mirabilite. The mirabilite was then filtered off and the filtrate was concentrated under reduced pressure to provide 4.03 g of a syrupy crude extract of YL-02107Q substance. For further purification, this crude extract was dissolved in 20 ml of chloroform and applied to a column prepared by packing a chromatographic tube having an outer diameter of 3 cm and a length of 110 cm with 550 ml of silica gel (Wakogel C-300, Wako Pure Chemical Industries, Ltd.) slurried with chloroform, and elution was carried out with chloroform-methanol (100:1). This eluate was collected in 20 g fractions using a fraction collector. For identification of the object compound, the fractions were subjected to chromatography using a silica gel plate (Kieselgel 60 $F_{254}$, 20 cm x 20 cm, Merck) and a developer system of chloroform-methanol (90:10). The plate was then sprayed with 10% sulfuric acid and heated at 110°C for 20 minutes for identification of the object compound YL-02107Q substance (Rf = 0.43). The object compound YL-02107Q substance was eluted in fraction No. 210 through fraction No. 300. Therefore, these fractions were pooled, concentrated to dryness under reduced pressure and dried overnight in a vacuum desiccator to provide 696 mg of a white powder of YL-02107Q substance with a purity of 92%.

Example 3

In 1 ml of methanol was dissolved 106.4 mg of the YL-02107Q substance with a purity of 92% as obtained in Example 2 and the solution in 0.2 ml portions was subjected to high performance liquid chromatography to obtain a pure product of YL-02107Q substance. The above HPLC was carried out by using a 2 cm x 25 cm STR-ODS-H column (Shimadzu Techno-Research, Inc.) and passing acetonitrile-tetrahydrofuran-methanol-water (31:12:19:38) as the eluent at the flow rate of 8 ml/min., with detection being made from the ultraviolet absorption at 220 nm. As a result, the object compound YL-02107Q substance was eluted at a retention time of 20 minutes and 40 seconds. This fraction was, therefore, collected. The purity of YL-02107Q substance contained in this fraction was tested by using a 4.6 mm x 250 mm high performance liquid chromatography column STR-ODS-H (preparative ODS-H) and the same solvent as above at the flow rate of 0.8 ml/min, with detection being made from the ultraviolet absorption at 220 nm. As a result, a peak of pure YL-02107Q substance was found at a retention time of 10 minutes and 51 seconds. The fraction containing this pure YL-02107Q substance amounted to 63 ml. This fraction was concentrated to 20 ml and shaken well with 50 ml of ethyl acetate to extract the object compound into the ethyl acetate layer. After this extraction, the water layer was separated and the residual ethyl acetate extract was thoroughly dehydrated by addition of mirabilite. After the mirabilite was filtered off, the filtrate was concentrated to dryness under reduced pressure. For further drying, the residue was dried overnight in a vacuum desiccator to provide 62.5 mg of pure YL-02107Q substance. For crystallization, this product was dissolved by addition of 0.2 ml of ethyl acetate and stirred with gradual addition of 0.6 ml of hexane. The mixture was allowed to stand in the refrigerator overnight for crystallization. The crystals thus formed were recovered by filtration and dried overnight in a vacuum desiccator to provide 53 mg of pure crystals as a white powder.

Example 4-a

A medium (pH 7.0) containing 1.0% of glucose, 2.0% of potato starch, 0.5% of yeast extract, 0.5% of polypeptone and 0.4% of calcium carbonate was prepared and distributed in 60 ml portions into 500 ml conical flasks followed by 20 minutes' sterilization at 120°C for use as a first-stage culture medium. This medium was inoculated with the mycelium of Kibdelosporangium sp. YL-02107Q picked from a good growth on Benett agar and shake culture was carried out at 28°C for 48 hours to prepare a first-stage seed culture.

The same medium as above was prepared, distributed in 18 ℓ portions into 30 ℓ tank fermenters and sterilized at 120°C for 30 minutes for use as a second-stage seed medium. This medium was inoculated with the above first-stage seed culture at the rate of 2% and incubated at 28°C for 72 hours at 200 rpm and an aeration rate of 30 ℓ/min. to prepare a second seed culture.

For production culture, 200 ℓ of a medium (pH 7.0) containing 1.0% of glucose, 2.0% of potato starch, 0.5% of soybean meal, 0.5% of feather meal and 0.4% of calcium carbonate was prepared in a 300 ℓ tank fermenter and sterilized at 120°C for 30 minutes. This medium was inoculated with the whole amount of the above second seed culture and incubated at 28°C for 6 days at 80 rpm and an aeration rate of 200 ℓ/min. Thereupon, the antibacterial activity of the object compound etc. against Bacillus subtilis ATCC 6633 became maximal. To the culture thus obtained was added Radiolite #600 (Showa Chemical Industries, Ltd.) and the mixture was stirred and filtered.

The culture filtrate (pH 7.8) thus obtained was adjusted to pH 7.0 with hydrochloric acid and passed through a column, 18 cm in outer diameter and 150 cm high, of Diaion HP-20 (Mitsubishi Kasei Corporation, 20 ℓ) to adsorb the object compound. The column was then rinsed well with 50 ℓ of distilled water and further washed with 40 ℓ of 25% acetone-water. Finally the object compound was eluted with 50 ℓ of 80% acetone-water. This eluate was concentrated under reduced pressure, adjusted to pH 7.0 and extracted with 40 ℓ of ethyl acetate. After phase separation, the water layer was discarded.

The ethyl acetate extract thus obtained was concentrated under reduced pressure to provide 430 g of a syrupy crude extract containing YL-02107Q substance and YL-02107Q-B through D substances. This crude extract was dissolved in 1000 ml of ethyl acetate and halves of the solution were applied to a couple of silica gel (Wakogel C-300, Wako Pure Chemical Industries, Ltd., 6ℓ) columns (9 x 150). Then, elution was carried out with ethyl acetate-methanol (100:0.5). Each eluate was collected in 500 ml fractions.

For identification of the object compound, each fraction was chromatographed using a silica gel plate (Kieselgel 60 $F_{254}$, 20 cm x 20 cm, Merck) and, as the developer, chloroform-methanol (9:1). The plate was then sprayed with 10% sulfuric acid and heated at 110°C for 20 minutes to confirm the object compound from the color reaction. The YL-02107Q substance was eluted in fraction Nos. 8 to 15. YL-02107Q-B through D were eluted in fraction Nos. 16 to 40. These fractions were concentrated to dryness under

reduced pressure and dried in a vacuum desiccator to provide 10 g of a mixture of YL-02107-Q-B through D.

This mixture of YL-02107Q-B through D was dissolved in 30 ml of chloroform and applied to a chromatographic column, 5 cm in outer diameter and 120 cm long, of 1 ℓ silica gel (Wakogel C-300, Wako Pure Chemical Industries, Ltd.) slurried with chloroform, elution being carried out with chloroform-methanol (100:3). This eluate was collected in 20 g fractions using a fraction collector. Identification of the object compound was carried out by silica gel thin-layer chromatography under the conditions described in Example 1 and by high performance liquid chromatography. The HPLC was carried out using a YMC-Pack-A-312 ODS (YMC Company, Ltd.) column, 4.6 mm x 150 mm, and, as the eluent, acetonitrile-methanol-tetrahydrofuran-water (20:30:10:40) at a flow rate of 1 ml/min., with detection being made from the ultraviolet absorption at 220 nm.

Example 4-b

The fraction containing YL-02107Q-B substance, fraction containing YL-02107Q-C substance and fraction containing YL-02107Q-D substance as obtained in Example 4-a were respectively concentrated to dryness under reduced pressure and dried in a vacuum desiccator overnight to provide 1.4 g, 1.2 g and 1.0 g of white powders, respectively.

Then, 400 mg each of these white powders were respectively dissolved in 4 ml of methanol and 0.4 ml aliquots thereof were subjected to high performance liquid chromatography to obtain the desired pure forms of YL-02107Q-B substance, YL-02107Q-C substance and YL-02107Q-D substance, respectively. The HPLC was carried out using a YMC-Pack-D-5 ODS (YMC Company, Ltd.) column, 2 cm x 25 cm, and, as the eluent, acetonitrile-methanol-tetrahydrofuran-water (20:30:10:40) at a flow rate of 10 ml/min., with detection being made from the ultraviolet absorption at 220 nm.

The respective fractions were concentrated and extracted with ethyl acetate. The ethyl acetate extract was dehydrated with mirabilite and filtered, and the filtrate was concentrated to dryness under reduced pressure. These residues were dried in a vacuum desiccator overnight to obtain 101 mg of YL-02107Q-B substance, 97 mg of YL-02107Q-C substance and 90 mg of YL-02107Q-D substance each as a pure white powder. The chemical structures and physicochemical properties of YL-02107Q substance and YL-02107Q-B through D substances are set forth hereunder.

YL-02107Q substance

Physicochemical properties

(1) Color and shape: A white crystalline powder
(2) Acidity, neutrality or basicity: neutral
(3) Solubility: Soluble in methanol, ethanol, acetone, ethyl acetate, benzene, toluene and chloroform but is only sparingly soluble in water and hexane.
(4) Melting point: 145.5 °C - 147.5 °C
(5) Optical rotation: $[\alpha]_D^{20}$ = -76.07 (c = 1%, methanol)

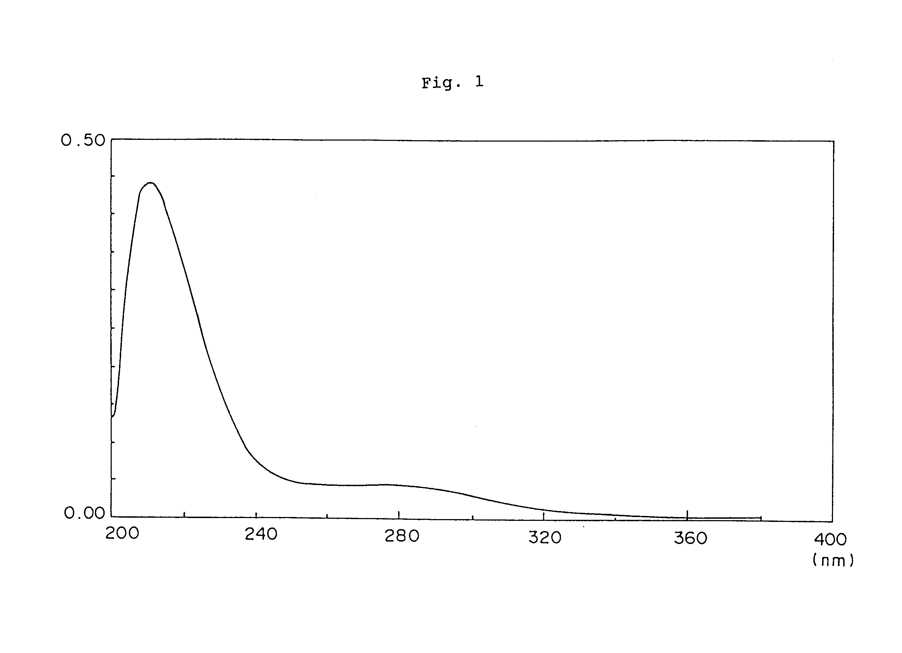

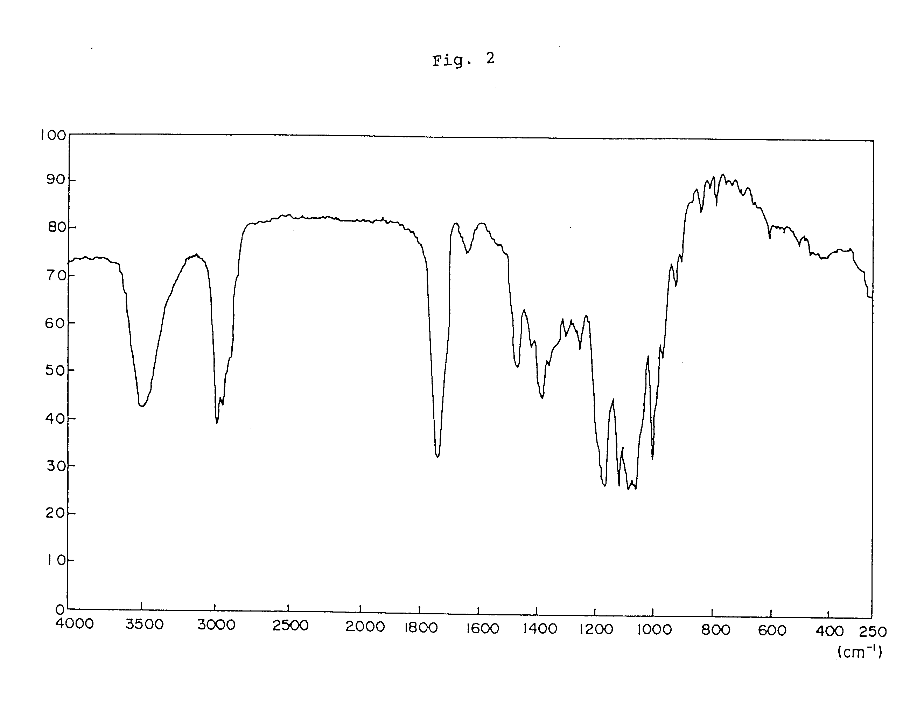

(6) Ultraviolet absorption spectrum: The ultraviolet absorption spectrum of YL-02107Q substance in methanol is presented in Fig. 1.
(7) Infrared absorption spectrum (KBr): Presented in Fig. 2
(8) Molecular weight: 963.1644
(9) Mass spectrum: 985 [M + Na] (FAB-Mass)
(10) Molecular formula: $C_{48}H_{82}O_{19}$
(11)

| Elemental analysis: (as $C_{48}H_{82}O_{19}\cdot 0.3\ H_2O$) | | | |
|---|---|---|---|
| | C(%) | H(%) | N(%) |
| Found | 59.43 | 8.49 | 0.00 |
| Calcd. | 59.52 | 8.60 | 0.00 |

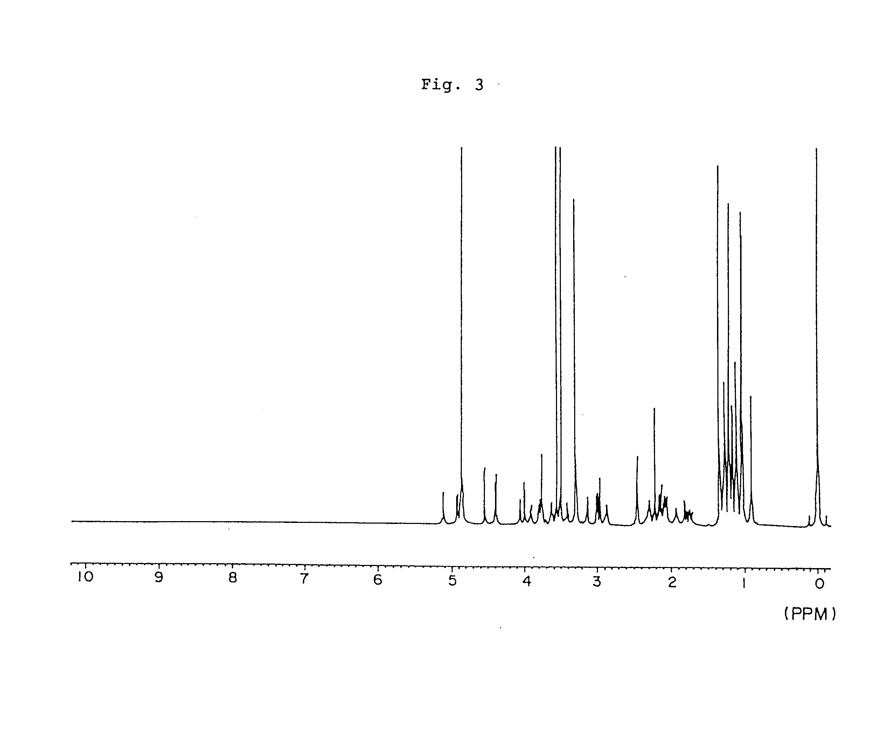

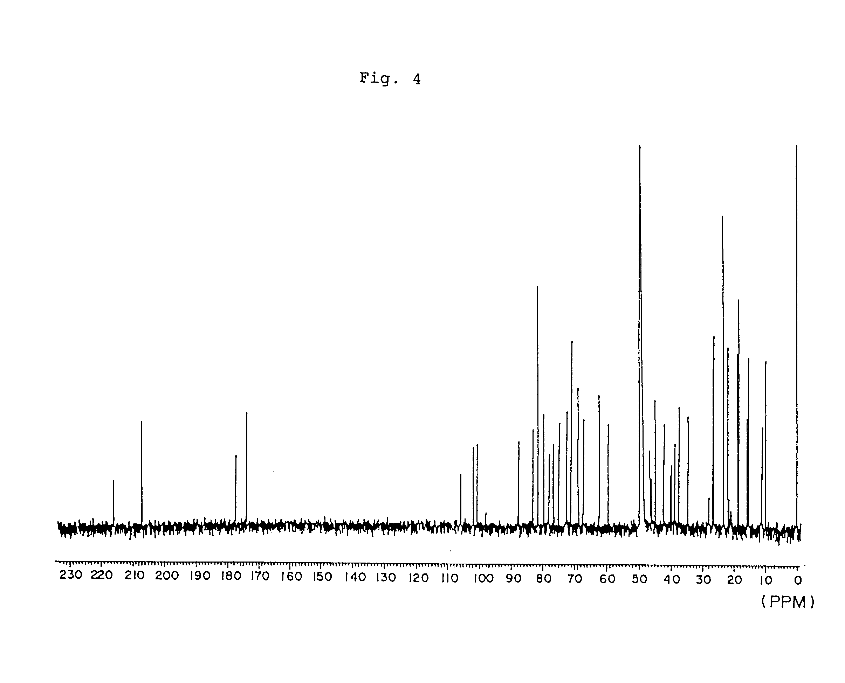

(12) $^1$H-NMR spectrum: The $^1$H-NMR spectrum of YL-02107Q substance is presented in Fig. 3.
(13) $^{13}$C-NMR spectrum: The $^{13}$C-NMR spectrum of YL-02107Q substance is presented in Fig. 4.

YL-02107Q-B substance

Physicochemical properties

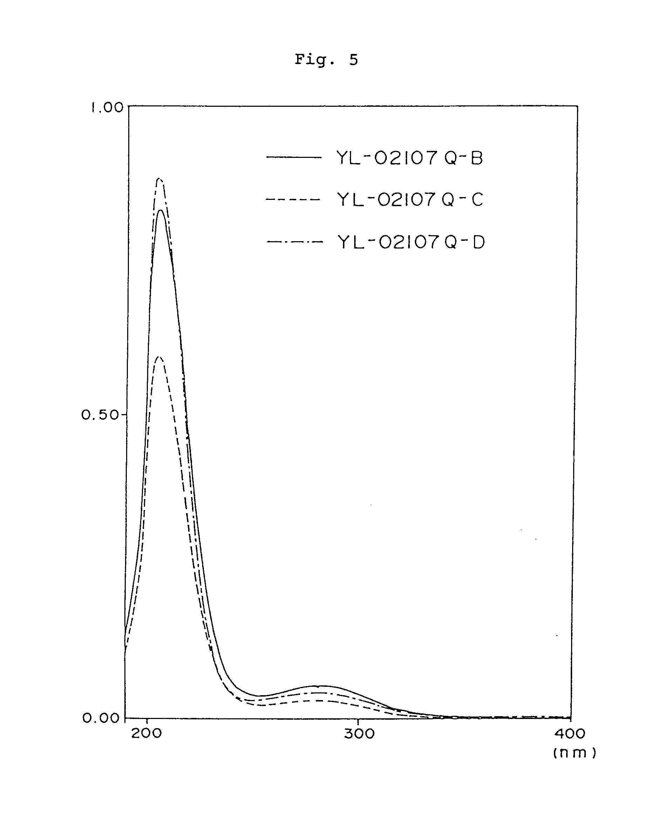

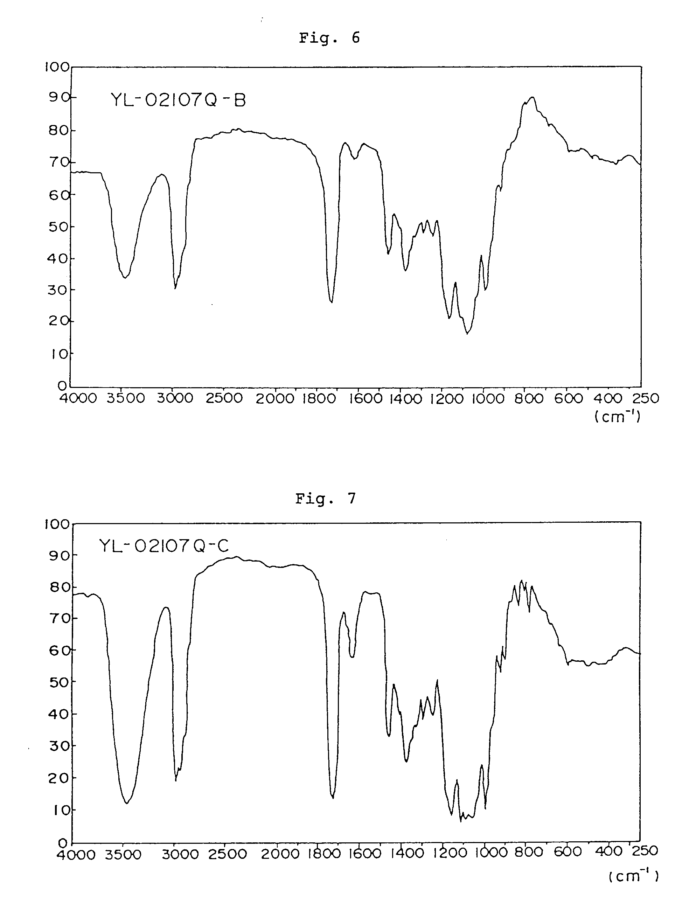

(1) Color and shape: A white powder
(2) Acidity, neutrality or basicity: neutral
(3) Solubility: Soluble in methanol, ethanol, acetone, ethyl acetate, benzene, toluene and chloroform but is only sparingly soluble in water and hexane.
(4) Melting point: 105.4 °C - 110.5 °C
(5) Optical rotation: $[\alpha]_D^{23}$ = -54.60 (c = 1%, methanol)
(6) Ultraviolet absorption spectrum: Presented in Fig. 5.
(7) Infrared absorption spectrum (KBr): Presented in Fig. 6
(8) Molecular formula: 949.141
(9) Mass spectrum: 971 m/z [M + Na] (FAB-Mass)
(10) Molecular weight: $C_{47}H_{80}O_{19}$
(11)

| Elemental analysis: (as $C_{47}H_{80}O_{19} \bullet nH_2O$) (n = 1.50) | | | | |
|---|---|---|---|---|
| | C(%) | H(%) | N(%) | 0(%) |
| Found | 57.83 | 8.57 | 0.00 | 33.60 |
| Calcd. | 57.91 | 8.38 | 0.00 | |

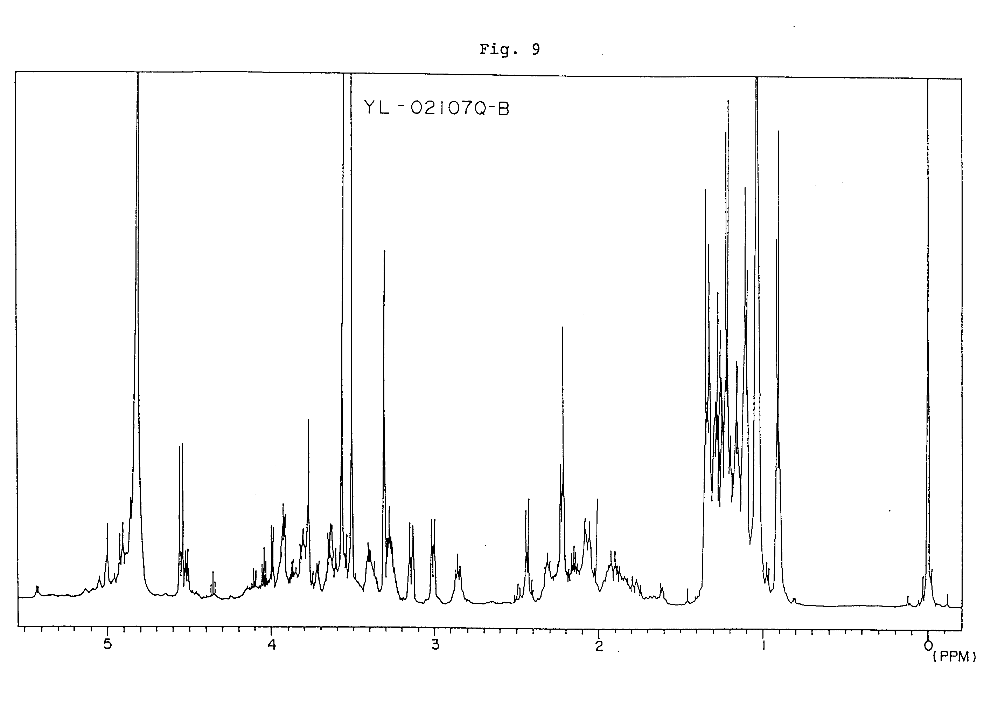

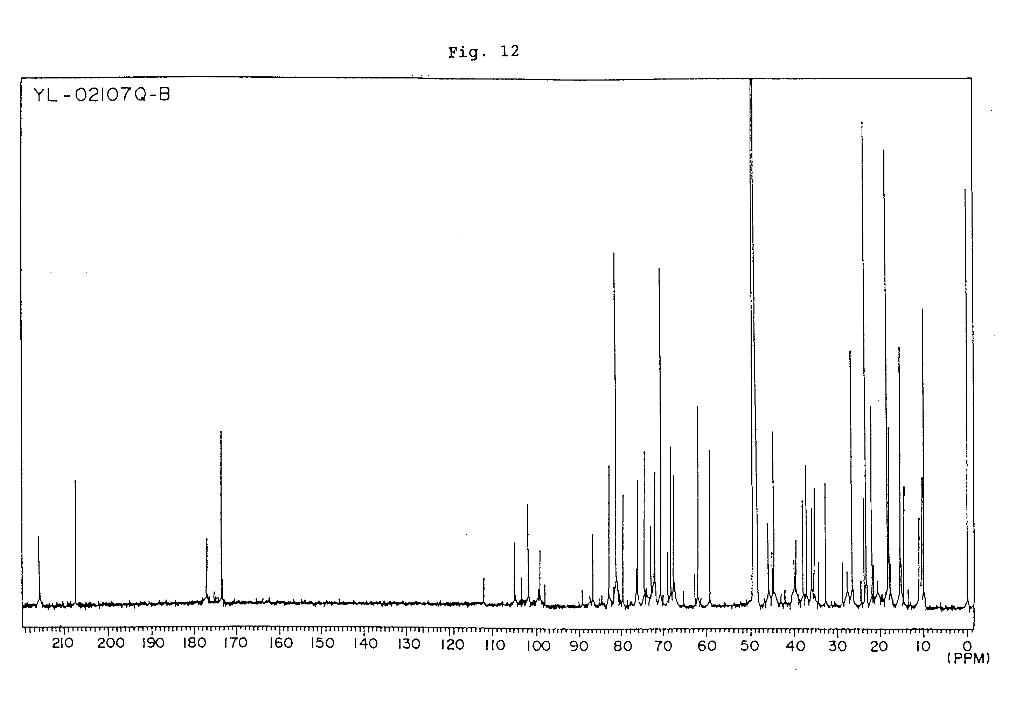

(12) $^{1}$H-NMR spectrum: Presented in Fig. 9.
(13) $^{13}$C-NMR spectrum: Presented in Fig. 12.
(14) Thin-layer chromatography:

| Stationary phase | Developing solvent | Rf value |
|---|---|---|
| Silica gel | Chloroform-methanol (9:1) (when the Rf value of YL-02107Q substance is 0.53) | 0.45 |
| Silica gel | Ethyl acetate-acetone (3:1) (when the Rf value of YL-02107Q substance is 0.45) | 0.35 |

(15) High performance liquid chromatography:

Column: YMC-Pack-A-312 ODS 4.6 mm x 150 mm (analytical)
Mobile phase: Acetonitrile-methanol-tetrahydrofuran-water (20:30:10:40)
Flow rate: 1 ml/min.
Detection: λ220 nm
Retention time (R.T.) = 13.9 min. (when the R.T. value of YL-02107Q substance = 20.1 min.)

YL-02107Q-C substance

Physicochemical properties

(1) Color and shape: A white powder
(2) Acidity, neutrality or basicity: neutral
(3) Solubility: Soluble in methanol, ethanol, acetone, ethyl acetate, benzene, toluene and chloroform but is only sparingly soluble in water and hexane.
(4) Melting point: 116.3 °C - 122.4 °C
(5) Optical rotation: $[\alpha]_D^{23}$ = -73.86 (c = 1%, methanol)
(6) Ultraviolet absorption spectrum: Presented in Fig. 5.
(7) Infrared absorption spectrum (KBr): Presented in Fig. 7
(8) Molecular weight: 979.167
(9) Mass spectrum: 1001.6 m/z [M + Na] (FAB-Mass)
(10) Molecular formula: $C_{48}H_{82}O_{20}$
(11)

| Elemental analysis: (as $C_{48}H_{82}O_{20} \cdot nH_2O$) (n = 1.50) | | | | |
|---|---|---|---|---|
| | C(%) | H(%) | N(%) | 0(%) |
| Found | 57.30 | 8.51 | 0.00 | 34.19 |
| Calcd. | 57.42 | 8.42 | 0.00 | |

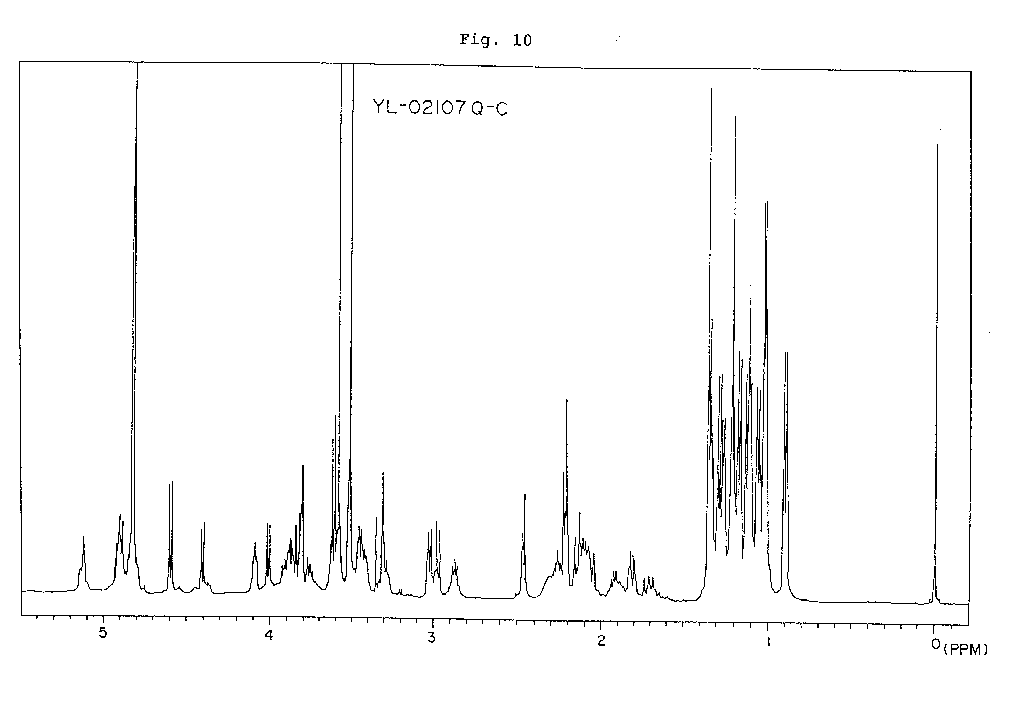

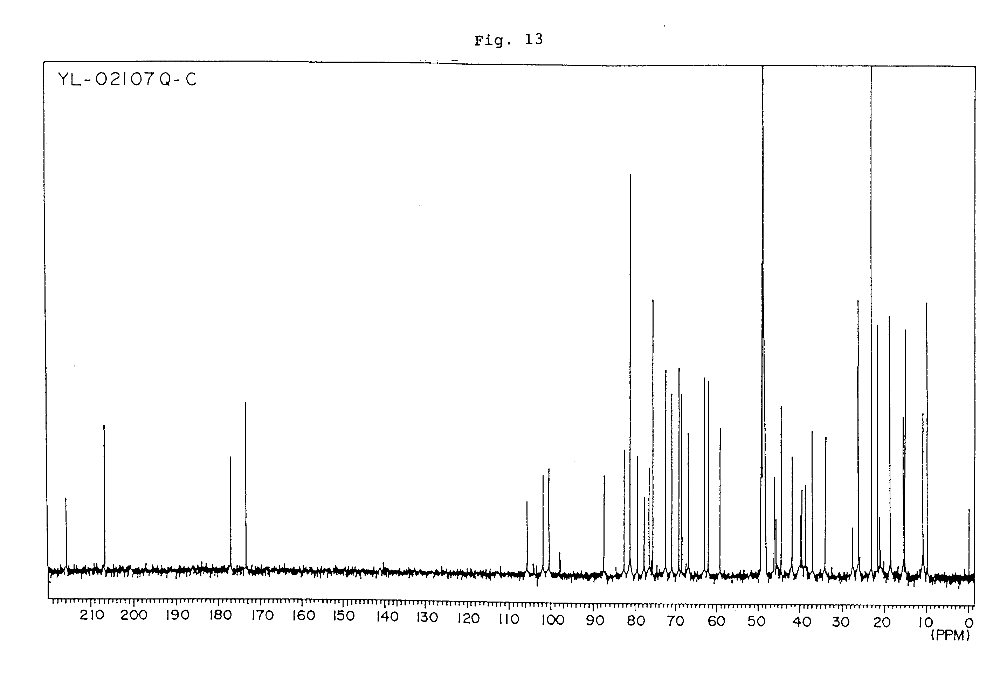

(12) $^1$H-NMR spectrum: Presented in Fig. 10.
(13) $^{13}$C-NMR spectrum: Presented in Fig. 13.
(14) Thin-layer chromatography:

| Stationary phase | Developing solvent | Rf value |
|---|---|---|
| Silica gel | Chloroform-methanol (9:1) (when the Rf value of YL-02107Q substance is 0.53) | 0.40 |
| Silica gel | Ethyl acetate-acetone (3:1) (when the Rf value of YL-02107Q substance is 0.45) | 0.30 |

(15) High performance liquid chromatography:
Column: YMC-Pack-A-312 ODS 4.6 mm x 150 mm (analytical)
Mobile phase: Acetonitrile-methanol-tetrahydrofuran-water (20:30:10:40)
Flow rate: 1 ml/min.
Detection: λ220 nm
Retention time (R.T.) = 11.1 min. (when the R.T. value of YL-02107Q substance = 20.1 min.)

YL-02107Q-D substance

Physicochemical properties

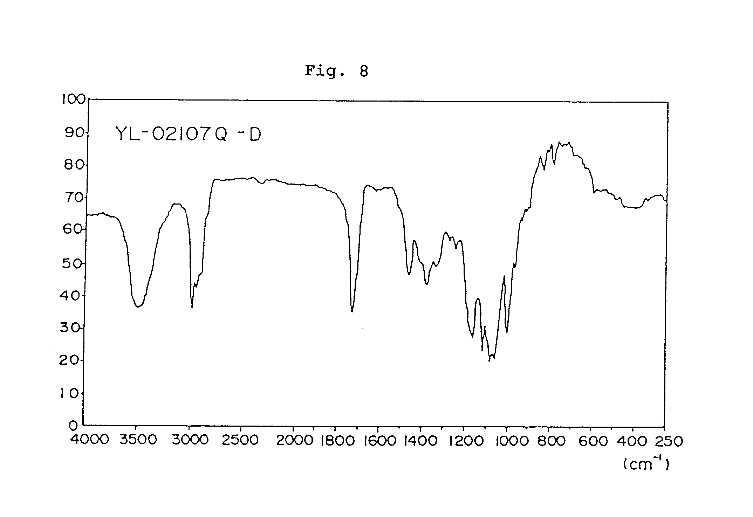

(1) Color and shape: A white powder
(2) Acidity, neutrality or basicity: neutral
(3) Solubility: Soluble in methanol, ethanol, acetone, ethyl acetate, benzene, toluene and chloroform but is only sparingly soluble in water and hexane.
(4) Melting point: 120.8°C - 127.2°C
(5) Optical rotation: $[\alpha]_D^{23}$ = -79.76 (c = 1%, methanol)
(6) Ultraviolet absorption spectrum: Presented in Fig. 5.
(7) Infrared absorption spectrum (KBr): Presented in Fig. 8
(8) Molecular weight: 879.050
(9) Mass spectrum: 901.2 m/z [M + Na] (FAB-Mass)
(10) Molecular formula: $C_{43}H_{74}O_{18}$
(11)

| Elemental analysis: (as $C_{43}H_{74}O_{18} \cdot nH_2O$) (n = 1.50) | | | | |
|---|---|---|---|---|
| | C(%) | H(%) | N(%) | O(%) |
| Found<br>Calcd. | 57.00<br>56.75 | 8.57<br>8.28 | 0.00<br>0.00 | 34.43 |

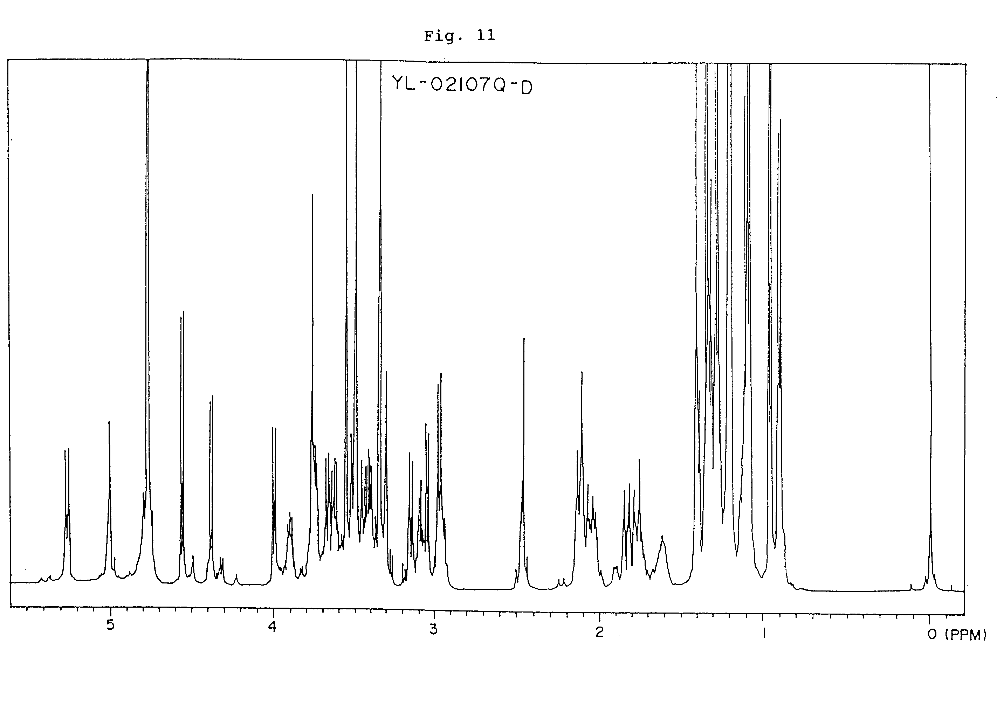

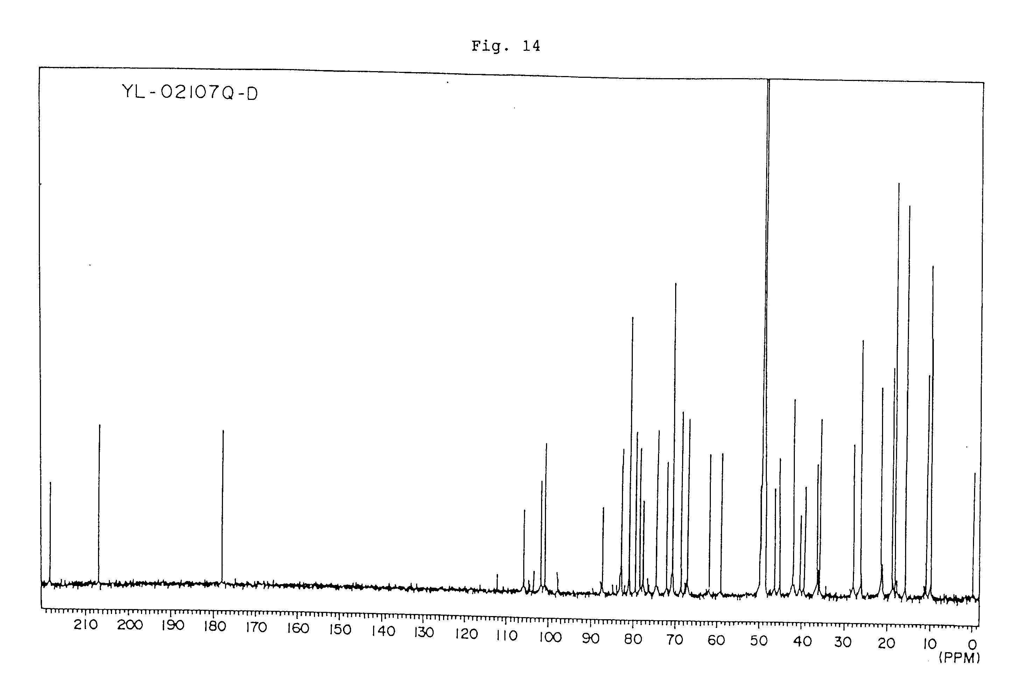

(12) $^1$H-NMR spectrum: Presented in Fig. 11.
(13) $^{13}$C-NMR spectrum: Presented in Fig. 14.
(14) Thin-layer chromatography:

| Stationary phase | Developing solvent | Rf value |
|---|---|---|
| Silica gel | Chloroform-methanol (9:1) (when the Rf value of YL-02107Q substance is 0.53) | 0.47 |
| Silica gel | Ethyl acetate-acetone (3:1) (when the Rf value of YL-02107Q substance is 0.45) | 0.38 |

(15) High performance liquid chromatography:

Column: YMC-Pack-A-312 ODS 4.6 mm x 150 mm (analytical)
Mobile phase: Acetonitrile-methanol-tetrahydrofuran-water (20:30:10:40)
Flow rate: 1 ml/min.
Detection: λ220 nm
Retention time (R.T.) = 6.6 min. (when the R.T. value of YL-02107Q substance = 20.1 min.)

Industrial Applicability

The novel macrolide compound which can be produced by the method of the present invention has potent activity against a broad spectrum of bacteria, particularly gram-positive bacteria, and has a very satisfactory pharmacokinetic profile. Furthermore, the macrolide compound of the present invention is of great use as the starting compound for the synthesis of other 14-membered ring macrolide compounds.

The antibacterial activity of the compound of this invention is shown below.

*In vitro* antibacterial activity (MIC)

The antibacterial spectrum of the macrolide compound of the present invention was determined by the agar plate dilution method using Müller-Hinton medium which is commonly used for this purpose.

Table 5

| Antibacterial spectra of macrolide compounds | | | | |
|---|---|---|---|---|
| Organisms | YL-02107Q substance (μg/ml) | Y-02107Q-B substance (μg/ml) | Y-02107Q-C substance (μg/ml) | Y-02107Q-D substance (μg/ml) |
| Staphylococcus aureus FDA 209P | 0.78 | - | - | - |
| Staphylococcus aureus FDA 209P JC-1 | - | 3.13 | 1.56 | 3.13 |
| Staphylococcus epidermidis IID866 | 0.78 | 3.13 | 1.56 | 1.56 |
| Streptococcus pyogenes Cook | 0.39 | 0.78 | 0.2 | 0.78 |
| Streptococcus pneumoniae IID552 | 3.13 | 50. | 6.25 | >50 |
| Enterococcus faecalis IID682 | 1.56 | 25. | 3.13 | >50 |
| Escherichia coli 0-1 | >100 | >50 | >50 | >50 |
| Klebsiella pneumoniae ATTCC10031 | >100 | >50 | >50 | >50 |
| Proteus vulgalis OX-19 | >100 | >50 | >50 | >50 |
| Serratia marcescens IID620 | >100 | >50 | >50 | >50 |
| Pseudomonas aeruginosa IID5142 | >100 | >50 | >50 | >50 |

The macrolide compound of the present invention can be administered orally or non-orally. Among dosage forms suited for administration are injections, tablets, capsules and syrups. These and other dosage forms can be manufactured in the routine manner using the conventional additives such as excipients, preservatives, stabilizers and so on.

The dosage varies with such factors as the route of administration, the patient's age, body weight and sex, and the drug or drugs used in combination. Generally, however, the daily dosage for adults may range from 200 to 600 mg, which can be administered in the single dose or in a few divided doses.

## Claims

**1.** A novel macrolide compound of the following chemical formula (I).

(wherein $R^1$ represents isovaleryloxy

$$\left( \begin{matrix} CH_3 \\ CH_3 \end{matrix} \!\!>\! CHCH_2\overset{\overset{\large O}{\|}}{C}O- \right)$$

or hydroxy; $R^2$ represents methyl or hydroxymethyl; $R^3$ represents methyl or hydrogen).

**2.** A macrolide compound as claimed in claim 1 wherein $R^1$ represents isovaleryloxy and $R^2$ and $R^3$ each represents methyl.

**3.** A microorganism of the genus Kibdelosporangium which has the ability to produce the macrolide compound claimed in claim 1.

**4.** A microorganism of the genus Kibdelosporangium as claimed in claim 3 which is Kibdelosporangium sp. YL-02107Q (FERM BP-3606).

0.50
0.00
200
240
280
320
360
400
(nm)

Fig. 1

Fig. 2

100
90
80
70
60
50
40
30
20
10
0
4000
3500
3000
2500
2000
1800
1600
1400
1200
1000
800
600
400
250
$(cm^{-1})$

0
1
2
3
4
5
6
7
8
9
10
(PPM)

Fig. 3

Fig. 4

0
10
20
30
40
50
60
70
80
90
100
110
120
130
140
150
160
170
180
190
200
210
220
230
(PPM)

Fig. 5

1.00
0.50
0.00
YL-02107 Q-B
YL-02107 Q-C
YL-02107 Q-D
200
300
400
(nm)

Fig. 6

YL-02107Q-B
100
90
80
70
60
50
40
30
20
10
0
4000 3500 3000 2500 2000 1800 1600 1400 1200 1000 800 600 400 250
(cm⁻¹)

Fig. 7

YL-02107Q-C
100
90
80
70
60
50
40
30
20
10
0
4000 3500 3000 2500 2000 1800 1600 1400 1200 1000 800 600 400 250
(cm⁻¹)

Fig. 8

YL-02107Q -D
100
90
80
70
60
50
40
30
20
10
0
4000 3500 3000 2500 2000 1800 1600 1400 1200 1000 800 600 400 250
(cm⁻¹)

Fig. 9

YL-02107Q-B
5
4
3
2
1
0
(PPM)

Fig. 10

YL-02107Q-C
5
4
3
2
1
0 (PPM)

YL-02107Q-D
0 (PPM)
1
2
3
4
5

Fig. 11

Fig. 12

YL-02107Q-B
0
10
20
30
40
50
60
70
80
90
100
110
120
130
140
150
160
170
180
190
200
210
(PPM)

YL-02107Q-C
0
10
20
30
40
50
60
70
80
90
100
110
120
130
140
150
160
170
180
190
200
210
(PPM)

Fig. 13

Fig. 14

YL-02107Q-D
210
200
190
180
170
160
150
140
130
120
110
100
90
80
70
60
50
40
30
20
10
0
(PPM)

# INTERNATIONAL SEARCH REPORT

International Application No PCT/JP91/01629

**I. CLASSIFICATION OF SUBJECT MATTER** (if several classification symbols apply, indicate all) [6]

According to International Patent Classification (IPC) or to both National Classification and IPC

Int. Cl[5] C07H17/08, C12N1/20, C12P19/62//A61K31/72, (C12P19/62, C12R1:01)

**II. FIELDS SEARCHED**

Minimum Documentation Searched [7]

| Classification System | Classification Symbols |
|---|---|
| IPC | C07H17/08, C12P19/62, C12N1/20 |

Documentation Searched other than Minimum Documentation to the Extent that such Documents are Included in the Fields Searched [8]

Chemical Abstracts Data Base (CA, REGISTRY)
Biological Abstracts Data Base (BIOSIS)

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | US, A, 4,543,334 (ptizer Inc.), September 24, 1985 (24. 09. 85) | 1-7 |
| A | Journal of the American Chemical Society, volume 102, no. 10, May 7, 1980 (07. 05. 80), B. Arnoux et al.: "23672RP, a New Macrolide Antibiotic from Streptomyces chryseus. Mass Spectrometry Study and x-ray Structure Determination," see p. 3605-3608 | 1-7 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| February 7, 1992 (07. 02. 92) | February 25, 1992 (25. 02. 92) |
| **International Searching Authority** | **Signature of Authorized Officer** |
| Japanese Patent Office | |